(19) Europäisches Patentamt
European Patent Office
Office européen des brevets

(11) EP 0 659 071 B1

(12) **EUROPEAN PATENT SPECIFICATION**

(45) Date of publication and mention of the grant of the patent:
**15.10.1997 Bulletin 1997/42**

(21) Application number: **93920410.3**

(22) Date of filing: **30.08.1993**

(51) Int. Cl.[6]: **A61K 7/50**

(86) International application number:
**PCT/US93/08137**

(87) International publication number:
**WO 94/05256 (17.03.1994 Gazette 1994/07)**

# (54) MILD, SUBSTANTIALLY COLORLESS SHAMPOO COMPOSITION

MILDE, IM WESENTLICHEN FARBLOSE SHAMPOOZUSAMMENSETZUNG

COMPOSITION DE SHAMPOOING DOUX SENSIBLEMENT INCOLORE

(84) Designated Contracting States:
**AT BE CH DE DK ES FR GB GR IE IT LI LU NL PT SE**

(30) Priority: **08.09.1992 US 941594**

(43) Date of publication of application:
**28.06.1995 Bulletin 1995/26**

(73) Proprietor: **THE PROCTER & GAMBLE COMPANY**
**Cincinnati, Ohio 45202 (US)**

(72) Inventor: **KALLA, Karen, Kay**
**Loveland, OH 45140 (US)**

(74) Representative: **Brooks, Maxim Courtney et al**
**Procter & Gamble**
**(Health & Beauty Care) Limited**
**Rusham Park,**
**Whitehall Lane**
**Egham, Surrey TW20 9NW (GB)**

(56) References cited:
**EP-A- 0 150 323**
**EP-A- 0 229 690**
**EP-A- 0 356 784**
**US-A- 5 227 156**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.14.16/3.4

EP 0 659 071 B1

## Description

The present invention Is related to shampoo compositions. More specifically, the present invention relates to mild, substantially colorless shampoos, especially those that are useful for application to the hair.

Mild shampoo compositions which provide low irritation to the skin are highly desirable. Conventional shampoos contain high levels of harsh anionic surfactants. These surfactants can provide excellent lathering and cleaning characteristics. However, they can also penetrate the skin and destroy its integrity. This results, at the very least, in rough and/or dry skin, and can ultimately lead to red, irritated skin. Ideal shampoo compositions should provide sufficient lathering and cleaning benefits to cleanse the hair while at the same time causing little or no irritation to the skin. This is particularly essential for shampoo compositions used on babies, small children, or adults with dry or sensitive skin.

Mild shampoo compositions, in general, are well known in the art having been taught in, for example, EP-A-0250181, published December 23, 1987; US-A-4,578,216, Fujii et al., issued March 25, 1986; US-A-4,726,915, Verdicchio, Issued February 23, 1988; GB-A-1,508,929, published April 26, 1978; EP-A-0160269, published November 6, 1985; US-A-4,435,300, Guth et al., issued March 6, 1984; US-A-4,426,310, Vernunica, issued January 17, 1984; US-A-3,950,417, Verdicchio et al., issued April 13, 1976; US-A-4,443,362, Guth et al., issued April 17, 1984; US-A-4,654,207, Preston, issued March 31, 1987; US-A-4,851,154, Grollier et al., issued July 25, 1989; US-A-4,292,212, Melby, issued September 29, 1981; and US-A-4,329,334, Su et al., issued May 11, 1982. These references teach the use of a wide variety of surfactant combinations to achieve mildness. These surfactants include mild anionic surfactants, such as ethoxylated alkyl sulfates, amphoteric surfactants, nonionic surfactants, and various combinations thereof.

In addition to the physical effect harsh shampoos can have on the skin, consumers are also influenced by aesthetic factors relating to the shampoo. For example, high lathering is associated with good cleaning. Also, clear and colorless products are associated with purity and mildness. It would be highly desirable to provide a mild shampoo which is both clear and colorless, and which can provide high levels of lather upon use.

Unfortunately, the formulation of mild shampoos is generally an exercise in trade-offs and compromises. Mild shampoos conventionally have relatively poor lathering and are not colorless. Clear colorless shampoos tend to be harsh to the skin, to be characterized by poor lather and/or to suffer from discoloration after extended storage periods.

Hence, it is an object of the present invention to provide a mild shampoo composition which is substantially colorless even over extended periods of storage, and which provides good lathering while at the same time providing good cleaning and being mild to the skin.

This and other objects will become readily apparent from the detailed description which follows.

Unless otherwise indicated, all percentages are calculated by weight of the total composition and all ratios are calculated on a weight basis.

The compositions and processes herein can comprise, consist essentially of, or consist of any of the critical and optional elements and steps described in this disclosure.

### SUMMARY OF THE INVENTION

The present invention provides a mild, substantially colorless shampoo composition that can provide good lathering characteristics which comprises:

(a) from 5% to 15%, by weight, of an alkyl ethoxylated sulfate anionic surfactant having an average degree of ethoxylation of at least about 2.0;
(b) from 2% to 8%, by weight, of an amphoteric surfactant selected from the group consisting of betaine surfactants, imidazoline surfactants, aminoalkanoate surfactants, and iminodialkanoate surfactants, and mixtures thereof;
(c) from 0.5% to 10%, by weight, of an N-acylamino acid surfactant, or salt thereof;
(d) at least 0.05%, by weight, of the composition, of carbonyl-containing perfume compounds; and
(e) water;

wherein said shampoo composition is substantially free of alkyl sulfate anionic surfactant, primary amines, and ammonia, and said shampoo composition has an absorbance value at 440nm in a 1.0cm path length of no greater than about 0.030 after storage at 38°C for 30 days and wherein the ratio of (a) to (b) is in the range of from 10:1 to 2:1 and wherein the alkyl ethoxylated sulfate anionic surfactant is a alkali or alkaline earth metal salt.

Although it is not intended to necessarily limit the invention by theory, it is believed that ammonia, which is typically introduced into the compositions in the form of ammonium ions, and/or primary amines react with carbonyl functionalities present in compounds found in most perfume formulations to form Schiff bases. These Schiff bases discolor the compositions. Insofar as it is desired to use conventional perfume formulations in the present shampoo compositions, the compositions hereof contain carbonyl-containing perfume compounds but must be essentially free of primary amines and ammonia. For purposed hereof, compositions substantially free of primary amines and ammonia includes

the protonated as well as unprotonated forms of the compounds.

Once ammonia and primary amines are eliminated from the compositions, it becomes possible to prepare substantially colorless shampoos. However, in order to formulate shampoos which are also mild and high lathering, it is desirable to use certain amphoteric surfactants which typically add some degree of color, usually a yellow tint, to the shampoo. Whereas this is not significant in the formulation of most shampoos (including the various-colored "clear" shampoos) it is undesirable in the forumlation of substantially colorless shampoos. Thus, it is also a necessary aspect of this invention that the amphoteric surfactants utilized in the present shampoo compositions be sufficiently colorless and free of primary amines and ammonia such that the shampoo composition itself remains substantially colorless.

The invention, including preferred embodiments thereof, is described in more detail in the Detailed Description of the Invention, which follows.

## DETAILED DESCRIPTION OF THE INVENTION

The essential as well as a variety of optional components of the compositions of the present invention are described below.

### Alkyl Ethoxylated Sulfate

The shampoo composition hereof comprises from 5% to 15%, preferably from 7% to 15%, of alkyl ethoxylated sulfate anionic surfactant.

Alkyl ethoxylated sulfate surfactants are well known in the art, and can be represented by the formula $RO(C_2H_4O)_xSO_3M$, wherein R is alkyl or alkenyl of from about 8 to about 24 carbon atoms, x is 1 to 12, preferably, and M is a water-soluble alkali or alkaline earth metal cation, preferably, sodium or potassium. The average degree of ethoxylated, i.e. the average value for x should be at least about 2.0.

Exemplary alkyl ethoxylated sulfates are condensation products of ethylene oxide and monohydric alcohols having from 8 to 24 carbon atoms. Preferably, R has from 10 to 18 carbon atoms. The alcohols can be derived from fats, e.g., coconut oil or tallow, or can be synthetic. Lauryl alcohol and straight chain alcohols derived from coconut oil are preferred herein. Such alcohols are typically reacted with from 2 to 12, preferably 2 to 6, more preferably about 3, molar proportions of ethylene oxide and the resulting mixture of molecular species having, for example, an average moles of ethylene oxide per mole of alcohol also within the above limits, is sulfated and neutralized.

Specific examples of alkyl ethoxylated sulfates which may be used in the present invention are the salts, especially sodium and/or potassium salts, of coconut alkyl triethylene glycol ethoxylated sulfate, tallow alkyl triethylene glycol ethoxylated sulfate, and tallow alkyl hexaoxyethylene sulfate. Typically the alkyl ether sulfates will comprise a mixture of individual compounds, said mixture preferably having an average alkyl chain length of from 10 to 16 carbon atoms, and an average degree of ethoxylation of from 2 to 6 moles of ethylene oxide. Especially preferred are narrow range alkyl ethoxylate sulfates such as those having ethoxylation levels primarily in the range of 2. to 6.

### Amphoteric Surfactant

The amphoteric surfactant will be present in the shampoo compositions hereof at levels of from 2.5% to 8% by weight. The amphoteric component hereof is selected from the group consisting of amphoteric betaine, imidazoline, aminoalkanoate, and iminodialkanoate surfactants. The ratio of the alkyl ethoxylated surfactant to the amphoteric surfactant will be from 10:1 to 2:1.

The imidazoline amphoteric surfactants hereof are depicted by Formula I:

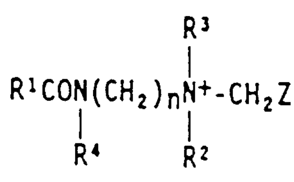

(I)

wherein $R^1$ is $C_8$ - $C_{22}$ alkyl or alkenyl, preferably $C_{12}$-$C_{16}$, $R^2$ is hydrogen or $CH_2CO_2M$, $R^3$ is $CH_2CH_2OH$ or $CH_2CH_2OCH_2CH_2COOM$, $R^4$ is hydrogen, $CH_2CH_2OH$, or $CH_2CH_2OCH_2CH_2COOM$, Z is $CO_2M$ or $CH_2CO_2M$, n is 2 or 3, preferably 2, M is hydrogen or a cation, such as alkali metal or alkaline earth metal. Examples of "alkali metal" include lithium, sodium, and potassium. Examples of "alkaline earth metal" include beryllium, magnesium, calcium, strontium, and barium. This type of surfactant is classified herein as an "imidazoline" amphoteric surfactant for convenience, although it should be recognized that it does not necessarily have to be derived, directly or indirectly, through an

imidazoline intermediate.

Suitable materials of this type are marketed under the tradename MIRANOL and are understood to comprise a complex mixture of species, and can exist in protonated and non-protonated species depending upon pH with respect to species that can have a hydrogen at $R^2$. All such variations and species are meant to be encompassed herein.

Preferred surfactants of Formula I are monocarboxylates and dicarboxylates. Examples of these materials include cocoamphocarboxypropionate, cocoamphocarboxypropionic acid, cocoamphocarboxyglycinate (alternately referred to as cocoamphodiacetate), and cocoamphoacetate (alternately, cocoamphomonoacetate).

Specific commercial products providing the amphoteric surfactant component of the present compositions include those sold under the trade names MIRANOL C2M CONC. N.P., MIRANOL C2M CONC. O.P., MIRANOL C2M SF, MIRANOL CM SPECIAL (Miranol, Inc.); ALKA-TERIC 2CIB (Alkaril Chemicals); AMPHOTERGE W-2 (Lonza, Inc.); MONATERIC CDX-38, MONATERIC CSH-32 (Mona Industries); REWOTERIC AM-2C (Rewo Chemical Group); and SCHERCOTERIC MS-2 (Scher Chemicals).

Suitable betaine surfactants hereof are depicted by compounds having the Formula (II):

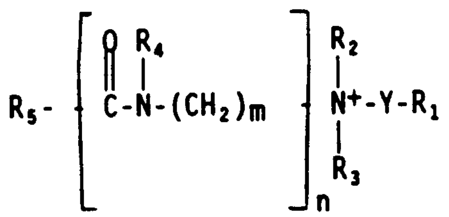

(II)

wherein:

$R_1$ is a member selected from the group consisting of
COOM and

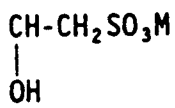

$R_2$ is lower alkyl or hydroxyalkyl;
$R_3$ is lower alkyl or hydroxyalkyl;
$R_4$ is a member selected from the group consisting of hydrogen and lower alkyl;
$R_5$ is higher alkyl or alkenyl;
Y is lower alkyl, preferably methyl;
m is an integer from 2 to 7, preferably from 2 to 3;
n is the integer 1 or 0;
M is hydrogen or a cation, as previously described, such as an alkali metal or alkaline earth metal.

The term "lower alkyl" or "hydroxyalkyl" means straight or branch chained, saturated, aliphatic hydrocarbon radicals and substituted hydrocarbon radicals having from one to about three carbon atoms such as, for example, methyl, ethyl, propyl, isopropyl, hydroxypropyl, hydroxyethyl, and the like. The term "higher alkyl or alkenyl" means straight or branch chained saturated (i.e., "higher alkyl") and unsaturated (i.e., "higher alkenyl") aliphatic hydrocarbon radicals having from about eight to about 20 carbon atoms such as, for example, lauryl, cetyl, stearyl, oleyl, and the like.

Examples of surfactant betaines of Formula II wherein n is zero which are useful herein include the alkylbetaines such as cocodimethylcarboxymethylbetaine, lauryldimethylcarboxymethylbetaine, lauryl dimethyl-alpha-carboxyethylbetaine, cetyldimethylcarboxymethylbetaine, lauryl-bis-(2-hydroxyethyl)carboxymethylbetaine, stearyl-bis-(2-hydroxypropyl)carboxymethylbetaine, oleyldimethyl-gamma-carboxypropylbetaine, lauryl-bis-(2-hydroxypropyl)-alpha-carboxyethylbetaine, etc. The sulfobetaines may be represented by cocodimethylsulfopropylbetaine, stearyldimethylsulfopropylbetaine, lauryl-bis-(2-hydroxyethyl)sulfopropylbetaine, and the like.

Amido betaines and amidosulfo betaine surfactants useful in the present invention are exemplified by compounds of Formula II wherein n is one but otherwise corresponding to the above examples. Examples of surfactant betaines of Formula II wherein n is one which are useful herein include the amidocarboxybetaines, such as cocoamidodimethylcarboxymethylbetaine, laurylamidodimethylcarboxymethylbetaine, cetylamidodimethylcarboxymethylbetaine, laurylamido-bis-(2-hydroxyethyl)-carboxymethylbetaine, cocoamido-bis-(2-hydroxyethyl)-carboxymethylbetaine, etc. The amido sulfobetaines may be represented by cocoamidodimethylsulfopropylbetaine, stearylamidodimethylsulfopropylbetaine, laurylamido-bis-(2-hydroxyethyl)-sulfopropylbetaine, and the like.

The preferred betaine in the present invention is a member selected from the group consisting of surfactant amidocarboxybetaines and amidosulfobetaines. More preferred betaines are the surfactant amidocarboxybetaines, particularly cocoamidodimethylcarboxymethylbetaines (cocomidopropylbetaine), such as those sold by Goldschmidt Co. under the trade name Tegobetaine L-7(R grade), and by Hoechst-Celanese under the trade name Genagen CAB. These most preferred betaines have the formula:

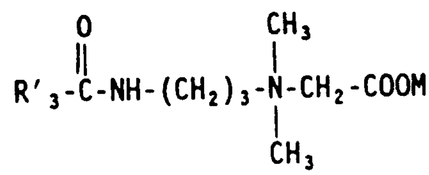

wherein $R'_3$ is selected from C8 to C18 alkyl radicals and M is hydrogen or a cation as defined above. In general, the preferred betaines hereof will have low levels of residual amide and sodium monochloroacetate.

Suitable aminoalkanoates and iminodialkanoates are represented by the Formulas (III) and (IV):

aminoalkanoates of the formula:

$$R\text{-}NH(CH_2)_nCOOM \qquad (III)$$ ;and

iminodialkanoates of the formula:

$$R\text{-}N[(CH_2)_mCOOM]_2 \qquad (IV)$$

wherein n and m are from 1 to 4, R is $C_8$ - $C_{22}$ alkyl or alkenyl, and M is hydrogen or alkali or an alkaline earth metal as previously described.

Examples of amphoteric surfactants falling within the aminoalkanoate formula include n-alkylamino-propionates and n-alkyliminodipropionates. Such material are sold under the tradename DERIPHAT by Henkel and MIRATANE by Miranol, Inc. Specific examples include N-lauryl-beta-amino propionic acid or salts thereof, and N-lauryl-beta-iminodipropionic acid (DERIPHAT 160C) or salts thereof, and mixtures thereof.

The amphoteric surfactant provided as a raw material for use in the shampoo compositions hereof must be sufficiently colorless such that it does not impact enough color to the total composition remains substantially colorless.

Amphoteric surfactants are often used in conventional shampoo compositions at a level and supplied in a form that tend to impart a degree of color in excess of that which is preferred for the compositions herein. The amphoteric surfactants utilized in the present compositions must be sufficiently colorless so that the overall absorbance value of the final product is within the limits set forth herein. Reduced color amphoteric surfactants raw materials can easily be produced by those skilled in the art, for instance, by the use of cleaner, purer raw materials and minimizing the period of time the surfactant or reaction mixture is exposed to elevated temperature during manufacture of the surfactant. Suitable surfactants are currently commercially available. e. g. Tegobetaine L-7 (R Grade) from Goldschmidt.

Preferably, the amphoteric surfactant, as well as the other surfactants added to the composition, will exhibit an absorbance value in a 30% aqueous solution at 440nm in a 1.0cm path length of no greater than about 0.1, more preferably no greater than about 0.05, most preferably no greater than about 0.035.

<u>N-Acylamino Acid Surfactant</u>

The shampoo compositions of the present invention comprise from 0.5% to 10%, preferably from 0.5% to 5%, more preferably from 1% to 5% by weight, of N-acyl amino acid surfactant.

N-acyl amino acid surfactants, for purposes hereof, include N-acyl hydrocarbyl acids and salts thereof, such as those represented by Formula V, as follows:

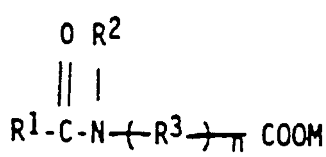 (V)

wherein: $R^1$ is a $C_8$-$C_{24}$ alkyl or alkenyl radical, preferably $C_{12}$-$C_{18}$; $R^2$ is -H, $C_1$-$C_4$ alkyl, phenyl, or $-CH_2COOM$, preferably $C_1$-$C_4$ alkyl, more preferably $C_1$-$C_2$ alkyl; $R_3$ is $-CR^4{}_2$- or $C_1$-$C_2$ alkoxy, wherein each $R^4$ independently is -H or $C_1$-$C_6$ alkyl or alkylester, and n is from 1 to 4, preferably 1 or 2; and M is -H or a cation as previously defined, preferably an alkali metal such as sodium or potassium.

A wide variety of N-acyl acid surfactants and their synthesis are described in Anionic Surfactants, Part II, Surfactant Science Series, Vol. VII, edited by Warner M. Linfield, Marcel Dekker, Inc. (New York and Basel), 1976; pp 581-617.

Especially preferred are compounds of Forumla V wherein $R^2$ is methyl and $R^3$ is $-CH_2$-, an n is 1, which are known as the N-acyl sarcosinates, and acids thereof. Specific examples include lauroyl sarcosinate, myristoyl sarcosinate, cocoyl sarcosinate, and oleoyl sarcosinate, preferably in their sodium and potassium salt forms.

For the purposes of the surfactants described herein, it should be understood that the terms "alkyl" or "alkenyl" include mixtures of radicals which may contain one or more intermediate linkages such as ether or polyether linkages or non-functional substitutents such as hydroxyl or halogen radicals wherein the radical remains of hydrophobic character.

Perfume

Perfumes are a highly desireable element of hair care compositions, since they can mask undesirable odors of other ingredients of the shampoo, as well as provide a pleasing aesthetic fragrance. Accordingly, perfume is an essential ingredient in the present compositions.

A wide variety of perfumes are known to those skilled in the art and are commercially available. The particular perfume used is largely a matter of choice. However the perfume should be used at a level effective for providing a noticeable aroma to the composition, or for masking undesired aroma of the composition.

In general, the compositions will comprise from 0.05% to 10% of a perfume compounds containing carbonyl functionalities, preferably from 0.1% to 7%, more preferably from 0.1% to 3% by weight. As used herein, perfume compound means aromatically active ingredients whereas "perfume" or "perfume component" includes the total perfume compounds and any accompanying perfume solvent.

Perfumes are made by those skilled in the art in a wide variety of fragrances and strengths. Typical perfumes are described in Arctander, Perfume and Flavour Chemicals (Aroma Chemicals), Vol. I and II (1969); and Arctander, Perfume and Flavour Materials of Natural Origin (1960).

Carbonyl-containing perfume compounds include aldehydes, ketones and esters. These materials can be aliphatic (saturated or unsaturated), aromatic, heterocyclic, or alicyclic. Generally they will have from about 6 to about 18 carbon atoms.

Aliphatic aldehyde perfume compounds include hexyl aldehyde, heptyl aldehyde, octyl aldehyde, decyl aldehyde, octyl aldehyde, decyl aldehyde, undecyl aldehyde, undecylinic aldehyde, lauric aldehyde, methyl monyl acetaldehyde, myristic aldelhyde, and nonyl aldehyde. Aromatic aldehydes include cinnamic aldehydes such as amyl cinnamic aldehyde and hexyl cinnamic aldehyde, anisic aldehyde, heliotropin, benzaldehyde, and vanillin. Heterocyclic aldehydes include piperonal and furan.

Aliphatic ketone perfume compounds include hexanone, heptanone, octanane, ionone, undecalactone, nonalactone (gamma-nonyl lactone), camphor, alpha-methyl ionone , (gamma-undecyl lactone) gamma-methyl ionone, and beta-methyl naphthyl ketone. Alicyclic ketones include, for example, amyl cyclohexanone. Aromatic ketones include beta-naphthyl methyl ketones, para-hydroxy phenolbutanone, laevo carvone, and musk ketone. Heterocyclic ketones include thujone, cedrenone, and exaltolide.

Aliphatic ester perfume compounds include acetates, butyrates, and formates, such as ethyl butyrate, ethyl formate, butyl acetate, isopropyl butyrate, ethyl formate, cis-3-hexenyl acetate, ethyl aceto acetate, and phenylethyl phenylacetate, carbonates such as methyl octine carbonate, and phthalates such as diethyl phthalate. Heterocyclic esters include cedryl formate and cedryl acetate. Aromatic esters include benzyl acetate, benzyl salicylate, cis-3-hexenyl acetate, cis-3-hexenyl salicylate, and methyl anthranilate. Other esters include alicyclic esters and aliphatic/alicyclic esters, such as 4 - tertiary butyl cyclohexyl acetate. Ester perfumes also include materials derived from floral materials and fruits, and from animal notes (e.g., natural isolates of civet, castoreum, and musk).

Perfume solvents are well known in the art, and the conventional ones can be used herein, e.g., dipropylene glycol, diethylene glycol, $C_1$-$C_6$ alcohols, etc.

The shampoo composition hereof will also comprise water. Generally, the composition will contain from 50% to 90% water, preferably 60% to 85% water, more preferably from 65% to 85% by weight.

The shampoo hereof is substantially free of alkyl sulfate surfactants since alkyl sulfates are relatively harsh to the skin. It is recognized that there will generally be some alkyl sulfate present as a result of it being present in commercially available alkyl ethoxylated sulfate raw materials. For example, commercially available alkyl (3) ethoxylated sulfate typically contains about 20% by weight alkyl sulfate; commercially available alkyl (2) ethoxylated sulfate, 25% to 40% alkyl sulfate. For purposes hereof, substantially free of alkyl sulfate means the compositions hereof should have an alkyl sulfate:alkyl ethoxylated sulfate (average degree of ethoxylation of 2.5 and above) weight ratio of no more than about 0.35,

preferably no more than about 0.30, more preferably no more than about 0.25. For alkyl ethoxylated sulfate with an average ethoxylation level of less than 2.5, the ratio should be no more than about 0.40, preferably no are than about 0.35, more preferably no more than about 0.30, most preferably no more than about 0.25. It is preferred that no additional amount of alkyl sulfate be added other than that which occurs inherently with the alkyl ethoxylated sulfate.

Narrow range ethoxylates can be used to lower the alkyl sulfate: alkyl ethoxylated sulfate weight ratio. "Narrow range ethoxylates" refer to alkyl ethoxylated sulfate surfactants that have been processed to reduce alkyl sulfates and, optionally, alkyl ethoxylated sulfates outside of the desired range of ethoxylation. The use of narrow range ethoxylates can be used to lower the alkyl sulfate:alkyl ethoxylated sulfate weight ratio, including to ratios as low as about 0.2 or even about 0.1, and less.

The present shampoo compositions are preferably substantially free of amide foam boosters such as fatty (e.g. $C_{12}$-$C_{20}$) mono-and di- alkanol amides, since these materials can be harsh to the skin and are not needed in the present compositions. "Substantially free of amide foam boosters" means the compositions hereof can contain no more than 1.0%, by weight of the amide foam booster, preferably no more than 0.5%, more preferably no more than 0.2% by weight. Most preferably, no amide foam booster is added.

As an essential aspect hereof, the shampoo compositions are substantially free of ammonia and primary amines. "Substantially free of ammonium ions and free primary amines" means that the compositions hereof can contain no more than 0.1%, by weight, of ammonia (including ammonium ions) and primary amines (including free amines and protonated primary amines), preferably no more than 0.05% by weight.

It is also preferred that no other ingredients that are unduly harsh to the skin be added to the shampoo compositions hereof.

The shampoo compositions of the present invention are substantially colorless, and can remain substantially colorless over prolonged periods of time. Substantially colorless, as used herein, means the shampoos are both clear and are characterized by lack of color. More specifically, the shampoo compositions hereof have an absorbance value at 440nm in a 1.0cm path length of no greater than about 0.030 after storage at 38°C for 30 days, preferably no greater than about 0.025. Absorbance value is measured utilizing a spectrophotometer in accordance with conventional techniques known in the art.

The compositions of the present invention can contain a wide variety of optional ingredients useful or known for use in the art for shampoo and other hair and skin cleansing compositions. Exemplary additional ingredients are described below.

Additional surfactants that can be used include other anionic, nonionic, and amphoteric surfactants, as well as zwitterionic and cationic surfactants.

A suitable class of anionic surfactants are the water-soluble, organic salts of the general formula:

$$R_1\text{-}SO_3\text{-}M$$

wherein $R_1$ is chosen from the group consisting of a straight or branched chain, saturated aliphatic hydrocarbon radical having from about 8 to about 24, preferably about 12 to about 18, carbon atoms; and M is a cation. Important examples are the salts of an organic sulfuric acid reaction product of a hydrocarbon of the methane series, including iso-, neo-, and n-paraffins, having 8 to 24 carbon atoms, preferably 12 to 18 carbon atoms and a sulfonating agent, e.g., $SO_3$, $H_2SO_4$, oleum, obtained according to known sulfonation methods, including bleaching and hydrolysis. Preferred are alkali metal sulfonated $C_{12\text{-}18}$ paraffins.

Additional examples of anionic surfactants which come within the terms of the present invention are the reaction products of fatty acids esterified with isethionic acid and neutralized with sodium hydroxide where, for example, the fatty acids are derived from coconut oil; sodium or potassium salts of fatty acid amides of methyl tauride in which the fatty acids, for example, are derived from coconut oil. Other anionic synthetic surfactants of this variety set forth in US-A-2,486,921; US-A-2,486,922; and US-A-2,396,278.

Still other anionic surfactants include the class designated as succinamates. This class includes such surface active agents as disodium N-octadecylsulfosuccinamate; tetrasodium N-(1,2-dicarboxyethyl)-N-octadecylsulfosuccinamate; diamyl ester of sodium sulfosuccinic acid; dihexyl ester of sodium sulfosuccinic acid; dioctyl esters of sodium sulfosuccinic acid.

Other suitable anionic surfactants utilizable herein are olefin sulfonates having 12 to 24 carbon atoms. The term "olefin sulfonates" is used herein to mean compounds which can be produced by the sulfonation of $\alpha$-olefins by means of uncomplexed sulfur trioxide, followed by neutralization of the acid reaction mixture in conditions such that any sulfones which have been formed in the reaction are hydrolyzed to give the corresponding hydroxy-alkanesulfonates. The sulfur trioxide can be liquid or gaseous, and is usually, but not necessarily, diluted by inert diluents, for example by liquid $SO_2$, chlorinated hydrocarbons, etc., when used in the liquid form, or by air, nitrogen, gaseous $SO_2$, etc., when used in the gaseous form.

Another class of anionic surfactants are the $\beta$-alkyloxy alkane sulfonates. These compounds have the following formula:

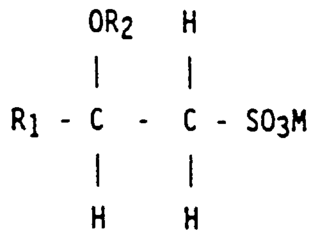

where $R_1$ is a straight chain alkyl group having from 6 to 20 carbon atoms, $R_2$ is a lower alkyl group having from about 1 (preferred) to 3 carbon atoms, and M is a water-soluble cation.

Many additional synthetic anionic surfactants are described in <u>McCutcheon's, Emulsifiers and Detergents, 1989 Annual</u>, published by M. C. Publishing Co. Also US-A-3,929,678, Laughlin et al., issued December 30, 1975, discloses many other anionic as well as other surfactant types. Soaps, of course, also fall within the scope of anionic detersive surfactants that can be used.

A wide variety of nonionic surfactants can be used. Nonionic surfactants include those broadly defined as compounds produced by the condensation of alkylene oxide groups (hydrophilic in nature) with an organic hydrophobic compound, which may be aliphatic or alkyl aromatic in nature. Examples of classes of nonionic surfactants are:

1. The polyethylene oxide condensates of alkyl phenols, e.g., the condensation products of alkyl phenols having an alkyl group containing from 6 to 20 carbon atoms, preferably from 6 to 12, in either a straight chain or branched chain configuration, with ethylene oxide, the said ethylene oxide being present in amounts equal to from 10 to 60 moles of ethylene oxide per mole of alkyl phenol. The alkyl substituent in such compounds may be derived from polymerized propylene, diisobutylene, octane, or nonane, for example.
2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from 40% to 80% polyoxyethylene by weight and having a molecular weight of from 5,000 to 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.
3. The condensation product of aliphatic alcohols having from about 8 to about 18 carbon atoms, in either straight chain or branched chain configuration, with ethylene oxide, e.g., a coconut alcohol ethylene oxide condensate having from 10 to 30 moles of ethylene oxide per mole of coconut alcohol, the coconut alcohol fraction having from 10 to 14 carbon atoms.
4. Long chain tertiary amine oxides corresponding to the following general formula:

$$R_1R_2R_3N \rightarrow O$$

wherein $R_1$ contains an alkyl, alkenyl or monohydroxy alkyl radical of from 8 to 18 carbon atoms, from 0 to 10 ethylene oxide moieties, and from 0 to 1 glyceryl moiety, and $R_2$ and $R_3$ contain from 1 to 3 carbon atoms and from 0 to 1 hydroxy group, e.g., methyl, ethyl, propyl, hydroxyethyl, or hydroxypropyl radicals. The arrow in the formula is a conventional representation of a semipolar bond. Examples of amine oxides suitable for use in this invention include dimethyl-dodecylamine oxide, oleyldi(2-hydroxyethyl) amine oxide, dimethyl-octylamine oxide, dimethyl-decylamine oxide, dimethyl-tetradecylamine oxide, 3,6,9-trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl) amine oxide, dimethylhexadecylamine oxide.
5. Long chain tertiary phosphine oxides corresponding to the following general formula:

$$RR'R''P \rightarrow O$$

wherein R contains an alkyl, alkenyl or monohydroxyalkyl radical ranging from 8 to 18 carbon atoms in chain length, from 0 to 10 ethylene oxide moieties and from 0 to 1 glyceryl moiety and R' and R'' are each alkyl or monohydroxyalkyl groups containing from 1 to 3 carbon atoms. The arrow in the formula is a conventional representation of a semipolar bond. Examples of suitable phosphine oxides are: dodecyldimethylphosphine oxide, tetradecyldimethylphosphine oxide, tetradecylmethylethylphosphine oxide. 3,6,9,-trioxaoctadecyldimethylphosphine oxide, cetyldimethylphosphine oxide, 3-dodecoxy-2-hydroxypropyldi(2-hydroxyethyl) phosphine oxide, stearyldimethylphosphine oxide, cetylethylpropylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl)phosphine oxide, tetradecylmethyl-2-hydroxypropylphosphine oxide, oleydimethylphosphine oxide, 2-hydroxydodecyldimethylphosphine oxide.

6. Long chain dialkyl sulfoxides containing one short chain alkyl or hydroxy alkyl radical of from 1 to 3 carbon atoms (usually methyl) and one long hydrophobic chain which include alkyl, alkenyl, hydroxy alkyl, or keto alkyl radicals containing from 8 to 20 carbon atoms, from 0 to 10 ethylene oxide moieties and from 0 to 1 glyceryl moiety. Examples include: octadecyl methyl sulfoxide, 2-ketotridecyl methyl sulfoxide, 3,6,9,-trixaoctadecyl 2-hydroxyethyl sulfoxide, dodecyl methyl sulfoxide, oleyl 3-hydroxypropyl sulfoxide, tetradecyl methyl sulfoxide, 3-methoxytridecyl methyl sulfoxide, 3-hydroxytridecyl methyl sulfoxide, 3-hydroxy-4-dodecoxybutyl methyl sulfoxide.

7. Polysorbates, e.g., sucrose esters of fatty acids. Such materials are described in US-A-3,480,616, e.g., sucrose cocoate (a mixture of sucrose esters of a coconut acid, consisting primarily of monoesters, and sold under the tradenames GRILLOTEN LSE 87K (TN) from RITA, and CRODESTA (TN) SL-40 from Croda).

8. Alkyl polysaccharide nonionic surfactants are disclosed in US-A-4,565,647, Llenado, issued January 21, 1986, having a hydrophobic group containing from 6 to 30 carbon atoms, preferably from 10 to 16 carbon atoms and a polysaccharide, e.g., a polyglycoside, hydrophilic group. The polysaccharide can contain from 1.0 to 10, preferably from 1.3 to 3, most preferably from 1.3 to 2.7 saccharide units. Any reducing saccharide containing 5 or 6 carbon atoms can be used, e.g., glucose, galactose and galactosyl moieties can be substituted for the glucosyl moieties. (Optionally the hydrophobic group is attached at the 2-, 3-, 4-, etc. positions thus giving a glucose or galactose as opposed to a glucoside or galactoside.) The intersaccharide bonds can be, e.g., between the one position of the additional saccharide units and the 2-, 3-, 4-, and/or 6-positions on the preceding saccharide units.

Optionally, and less desirably, there can be a polyalkyleneoxide chain joining the hydrophobic moiety and the polysaccharide moiety. The preferred alkyleneoxide is ethylene oxide. Typical hydrophobic groups include alkyl groups, either saturated or unsaturated, branched or unbranched containing from 8 to 18, preferably from 10 to 16, carbon atoms. Preferably, the alkyl group is a straight chain saturated alkyl group. The alkyl group can contain up to about 3 hydroxy groups and/or the polyalkyleneoxide chain can contain up to about 10, preferably less than 5, alkylene moieties. Suitable alkyl polysaccharides are octyl, nonyldecyl, undecyldodecyl, tridecyl, tetradecyl, pentadecyl, hexadecyl, heptadecyl, and octadecyl, di-, tri-, tetra-, penta-, and hexaglucosides, galactosides, lactosides, glucoses, fructosides, fructoses and/or galactoses. Suitable mixtures include coconut alkyl, di-, tri-, tetra-, and pentagluscosides and tallow alkyl, tetra-, penta-, and hexaglucosides.

9. Polyethylene glycol (PEG) glyceryl fatty esters, as depicted by the formula $RC(O)OCH_2CH(OH)CH_2(OCH_2CH_2)_nOH$ wherein n is from 5 to 200, preferably from 20 to 100, more preferably from 30 to 85, and RC(O)- is an ester wherein R comprises an aliphatic radical having from 7 to 19 carbon atoms, preferably from 9 to 17 carbon atoms, more preferably from 11 to 17 carbon atoms, most preferably from 11 to 14 carbon atoms. The combinations of n from 20 to 100, with $C_{12}$-$C_{18}$, preferably $C_{12}$-$C_{15}$ fatty esters, for minimized adverse effect on foaming, is preferred.

Suitable glyceryl fatty ester portions of these surfactants include glyceryl cocoate, glyceryl tallowate, glyceryl palmate, glyceryl stearate, glyceryl laurate, glyceryl oleate, glyceryl ricinoleate, and glyceryl fatty esters derived from triglycerides, such as palm oil, almond oil, and corn oil.

Other surfactants that can be used include soluble cationic surfactants, such as quaternary ammonium surfactants, and other amphoteric and zwitterionic surfactants known to those in the art.

An optional component hereof is a soluble conditioning agent suitable for conditioning hair or skin.

Suitable conditioners include, for example, soluble polyether siloxane copolymer, such as a polypropylene oxide modified dimethylpolysiloxane (e.g., Dow Corning DC-1248), although ethylene oxide or mixtures of ethylene oxide and propylene oxide may also be used. The ethylene oxide and polypropylene oxide level must be sufficiently high to provide solubility in water and the composition hereof.

The shampoo compositions can also contain one or more antidandruff agents. Soluble antidandruff agents should be used.

Hydroxypyridone salts are disclosed in *Cosmetics and Drug Preservation, Principles and Practice*, p 742 (edited by J. Kabura, 1984). Hydroxypyridone salts used herein include those generally described as 1-hydroxy-4-methyl-(1H)-pyridones having an aliphatic or aromatic moiety (R) at the 6 position thereof, wherein R has a factor of at least 1:3, preferably from 2 to 6, more preferably from 3 to 5.5. The factor is a measure of the lipophilicity/ hydrophilicity of the substituent and is defined in detail in the paper by W. Dittmar, E. Druckrey and H. Urbach, *J. Med. Chem.* 17(7), 753-6 (1974) and references cited therein.

In structural terms, preferred R substituents are selected from linear and branched $C_3$-$C_{11}$, preferably $C_6$-$C_{11}$, alkyl and alkenyl groups, $C_5$-$C_8$ cycloalkyl groups, and $C_5$-$C_8$, perferably $C_6$, aryl groups. The cyclic moieties, discussed above, can also be substituted with one or more alkyl or alkenyl groups up to $C_4$. The R groups can further be substituted with halogen atoms. Of the above, preferred R moieties are cyclohexyl and 2,4,4-trimethyl pentyl, the latter being highly preferred.

The above mentioned compounds can be used both in the free form and as salts, for example, salts with organic

bases or inorganic cations. Low molecular weight alkanolamines are especially preferred organic bases. The preferred hydroxypyridone salt for use herein is monoethanolamine salt known as piroctone olamine or Octopirox; see *Cosmetic and Drug Preservation, supra.*

The compositions herein can contain a variety of other non-essential optional components. Such optional ingredients include, for example, preservatives such as benzyl alcohol, methyl paraben, propyl paraben and imidazoiidinyl urea; cationic conditioning agents, including both cationic conditioning surfactant and cationic conditioning polymers; fatty alcohols; block polymers of ethylene oxide and propylene oxide such as Pluronic F88 offered by BASF Wyandotte; viscosity modifers, such as sodium chloride, sodium sulfate, and xylene sulfonate salts; propylene glycol; polyvinyl alcohol; ethyl alcohol; foam boosters such as Polyquaternium-10 (an industry term designated by The Cosmetic, Toiletry and Fragrance Association (CTFA) for the polymeric quaternary ammonium salt of hydroxyethyl cellulose reacted with trimethyl ammonium substituted epoxide), commercially available from Union Carbide Corp. (Danbury, Connecticut, USA) under their UCARE POLYMER JR series of materials, e.g., UCARE POLYMER JR-30M, JR-125 and JR-400; additional pH adjusting agents for the final shampoo composition such as citric acid, succinic acid, phosphoric acid, sodium hydroxide, sodium carbonate, etc.; perfumes; dyes; and sequestering acents such as disodium ethylenediamine tetraacetate.

Another type of suspending agent that can be used is carboxyvinyl polymer. Preferred polymers are copolymers of acrylic acid crosslinked with polyallylsucrose as described in US-A-2,798,053, Brown, issued July 2, 1957. These polymers are provided by B. F. Goodrich Company as, for example, Carbopol 934, 940, 941, and 956.

Other materials can also be used as suspension agents, including those that can impart a gel-like viscosity to the composition, such as water soluble or colloidally water soluble polymers like cellulose ethers (e.g., hydroxyethyl cellulose), guar gum, polyvinyl alcohol, polyvinyl pyrrolidone, hydroxypropyl guar gum, starch and starch derivative, and other thickeners, viscosity modifiers, gelling agents, etc. Mixtures of these materials can also be used.

Another type of suspending agent that can be used is xanthan gum. Shampoo compositions utilizing xanthan gum as a suspending agent for the silicone hair conditioning component are described in US-A-4,788,006, Bolich and Williams, issued November 29, 1988. Xanthan gum is biosynthetic gum material that is commercially available.

Optional ingredients are typically used at levels of from 0.01% to 10% by weight of the composition, This list of optional ingredients is not meant to be inclusive, as other optional components can be utilized.

The pH of the compositions for direct use for cleaning applications is preferably in the range of from 2 to 10, more preferably from 4 to 8, most preferably from 5 to 8.

## METHOD OF USE

The present compositions are used in a conventional manner for cleaning hair. An effective amount of the composition for cleaning hair, typically, from 1 g to 20 g of the composition, is applied to hair that has preferably been wetted, generally with water, and then rinsed out. Application to the hair typically includes working the composition through the hair such that most or all of the hair is contacted with the composition.

## EXAMPLES

The following Examples further describe and demonstrate the preferred embodiments within the scope of the present invention. The Examples are given solely for the purpose of illustration.

## EXAMPLES I-XIV

The following examples exemplify shampoo compositions of the present invention.

| Component (active wt.%) | I | II | III |
|---|---|---|---|
| Sodium Laureth-3 Sulfate (%)[1] | 10 | 10 | 13.5 |
| Cocoamidopropyl Betaine (%)[2] | 5 | 5 | 3.5 |
| Sodium Lauroyl Sarcosinate (%)[3] | 4.5 | 1.5 | 3.5 |
| Cocoamphodiacetate (%)[4] | - | - | - |
| Lauroiminodiacetate (%)[5] | - | - | - |
| Lauroiminodiproprionate (%)[6] | - | - | - |
| Dimethicone Copolyol (%)[7] | - | - | - |
| Polyol alkoxyester(%)[8] | - | - | - |
| Piroctone olamine (%) | - | - | - |
| Perfume (%)[9] | 0.3 | 0.3 | 0.3 |
| DMDM Hydantoin (%) | 0.37 | 0.37 | 0.37 |
| EDTA (%) | 0.1 | 0.1 | 0.1 |
| Citric Acid | 0.2 | 0.2 | 0.2 |
| NaCl | 2 | 1 | 1 |
| Water | q.s. | q.s. | q.s. |

| Component (active wt.%) | IV | V | VI | VII | VIII |
|---|---|---|---|---|---|
| Sodium Laureth-3 Sulfate (%)[1] | 13 | 12.8 | 13.5 | 13.5 | 13.5 |
| Cocoamidopropyl Betaine (%)[2] | 5 | 6 | - | - | - |
| Sodium Lauroyl Sarcosinate (%)[3] | 1 | 2 | 3.5 | 3.5 | 3.5 |
| Cocoamphodiacetate (%)[4] | - | - | 3.5 | - | - |
| Lauroiminodiacetate (%)[5] | - | - | - | 3.5 | - |
| Lauroiminodiproprionate (%)[6] | - | - | - | - | 3.5 |
| Dimethicone Copolyol (%)[7] | - | - | - | - | - |
| Polyol alkoxyester(%)[8] | - | - | - | - | - |
| Piroctone olamine (%) | - | - | - | - | - |
| Perfume (%)[9] | 0.3 | 0.3 | 0.3 | 0.3 | 0.3 |
| DMDM Hydantoin (%) | 0.37 | 0.37 | 0.37 | 0.37 | 0.37 |
| EDTA (%) | 0.1 | 0.1 | 0.1 | 0.1 | 0.1 |
| Citric Acid | 0.2 | 0.2 | - | - | - |
| NaCl | 1 | 1 | 1 | 1 | 1 |
| Water | q.s. | q.s. | q.s. | q.s. | q.s. |

| Component (active wt.%) | IX | X | XI |
| --- | --- | --- | --- |
| Sodium Laureth-3 Sulfate (%)[1] | 10 | 10 | 10 |
| Cocoamidopropyl Betaine (%)[2] | 5 | 5 | 5 |
| Sodium Lauroyl Sarcosinate (%)[3] | 4.5 | 4.5 | 4.5 |
| Cocoamphodiacetate (%)[4] | - | - | - |
| Lauroiminodiacetate (%)[5] | - | - | - |
| Lauroiminodiproprionate (%)[6] | - | - | - |
| Dimethicone Copolyol (%)[7] | 0.5 | - | - |
| Polyol alkoxyester(%)[8] | - | 1.5 | - |
| Piroctone olamine (%) | - | - | 1.0 |
| Perfume (%)[9] | 0.3 | 0.3 | 0.3 |
| DMDM Hydantoin (%) | 0.37 | 0.37 | 0.37 |
| EDTA (%) | 0.1 | 0.1 | 0.1 |
| Citric Acid | 0.2 | 0.2 | 0.2 |
| NaCl | 2 | - | 1 |
| Water | q.s. | q.s. | q.s. |

| Component (active wt.%) | XII | XIII | XIV |
| --- | --- | --- | --- |
| Sodium Laureth-3 Sulfate (%)[1] | 11 | 11 | 10 |
| Cocoamidopropyl Betaine (%)[2] | 5.5 | 5.5 | 5 |
| Sodium Lauroyl Sarcosinate (%)[3] | 1.6 | 1.6 | 0.5 |
| Cocoamphodiacetate (%)[4] | - | - | - |
| Lauroiminodiacetate (%)[5] | - | - | - |
| Lauroiminodiproprionate (%)[6] | - | - | - |
| Dimethicone Copolyol (%)[7] | - | - | - |
| Polyol alkoxyester(%)[8] | - | - | - |
| Piroctone olamine (%) | - | 1.0 | - |
| Perfume (%)[9] | 0.3 | 0.3 | 0.3 |
| DMDM Hydantoin (%) | 0.37 | 0.37 | 0.37 |
| EDTA (%) | 0.1 | 0.1 | 0.1 |
| Citric Acid | 0.2 | - | 0.2 |
| NaCl | 1 | 1 | 1 |
| Water | q.s. | q.s. | q.s. |

1 Absorbance value of no greater than about 0.025.

2 Absorbance value of no greater than about 0.035.

3 Absorbance value of no greater than about 0.025.

4 Absorbance value of no greater than about 0.035.

5 Absorbance value of no greater than about 0.035.

6 Absorbance value of no greater than about 0.035.

7 DOW 190, from Dow Chemical USA (Midland, Michigan).

8 CROTHIX, from Croda, Inc. (New York, New York).

9 Total perfume weight percent, containing about 75%, by weight of the total perfume, of carbonyl-containing perfume compounds.

The compositions are prepared as follows. The ethoxylated sulfate, sarcosinate, and amphoteric surfactant are mixed together in a tank and heated to 55°C. The EDTA is then dissolved in the mixture. As applicable, the piroctone olamine, dimethicone copolyol, and polyol alkoxyester are added to the composition with stirring. The perfume and DMDM Hydantoin are added and stirred in next. Finally the NaCl and citric acid are added and the mixture is stirred.

The compositions of the Examples can provide excellent in-use hair cleaning, lather, mildness and are substantially colorless after 30 days at 38°C.

## Claims

1. A mild, substantially colorless shampoo composition characterized in that it comprises:

   (a) from 5% to 15%, by weight, of an alkyl ethoxylated sulfate anionic surfactant having an average degree of ethoxylation of at least 2.0;
   (b) from 2.5% to 8% by weight of an amphoteric surfactant selected from the group consisting of betaine surfactants, imidazoline surfactants, aminoalkanoate surfactants, and iminodialkanoate surfactants, and mixtures thereof;
   (c) from 0.5% to 10%, by weight of an N-acylamino acid surfactant, or salt thereof;
   (d) at least 0.05%, by weight of the composition, of carbonyl-containing perfume compounds; and
   (e) water;

   wherein said shampoo composition is substantially free of alkyl sulfate anionic surfactant, primary amines, and ammonia, and said shampoo composition has an absorbance value at 440nm in a 1.0cm path length of no greater than 0.030 after storage at 38°C for 30 days and wherein the weight ratio of (a) to (b) is in the range of from 10:1 to 2:1 and wherein (a) is a alkali or alkaline earth metal salt

2. A shampoo composition according to Claim 1, wherein said amphoteric surfactant is selected from the group consisting of

   (i) betaine surfactants of the formula:

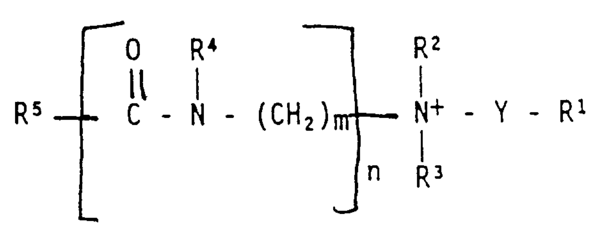

wherein $R_1$ is a member selected from the group consisting of
COOM and

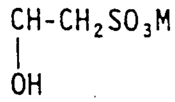

$R_2$ is lower alkyl or hydroxyalkyl;
$R_3$ is lower alkyl or hydroxyalkyl;
$R_4$ is a member selected from the group consisting of hydrogen and lower alkyl;
$R_5$ is higher alkyl or alkenyl;
Y is lower alkyl, preferably methyl;
m is an integer from 2 to 7;
n is the integer 1 or 0;
M is hydrogen or a cation;

(ii) amphoteric surfactant having the formula

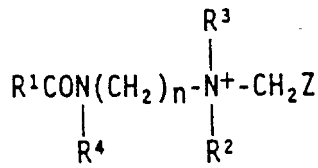

wherein $R^1$ is $C_8$-$C_{22}$ alkyl or alkenyl, $R^2$ is hydrogen or $CH_2CO_2M$, $R^3$ is $CH_2CH_2OH$ or $CH_2CHOCH_2CH_2COOM$, $R^4$ is hydrogen, $CH_2CH_2OH$, or $CH_2CH_2OCH_2CH_2COOM$, Z is $CO_2M$ or $CH_2CO_2M$, n is 2 or 3, and M is hydrogen or a cation; and
(iii) aminoalkanoates of the formula:

$$R\text{-}NH(CH_2)_nCOOM$$ ;and

iminodialkanoates of the formula:

$$R\text{-}N[(CH_2)_mCOOM]_2$$

wherein n and m are from 1 to 4, R is $C_8$ - $C_{22}$ alkyl or alkenyl, and M is hydrogen or alkali or alkaline with metal as previously described.
(iv) and mixtures thereof.

**3.** A shampoo composition as in Claim 1 or 2, wherein said N-acyl amino acid surfactant, or salt thereof, is of the formula:

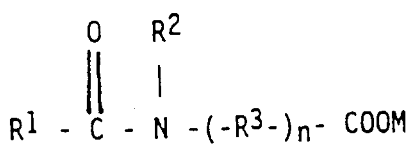

wherein: $R^1$ is $C_8$-$C_{24}$ alkyl or alkenyl :
$R^2$ is -H, $C_1$-$C_4$ alkyl, phenyl, or $-CH_2COOM$;
$R^3$ is $-C-R^4{}_2$; n is from 1 to 4; $R^4$ is H, $C_1$-$C_6$ alkyl, or $C_1$-$C_6$ alkylester; and M is H or a cation, or a mixture thereof.

**4.** A shampoo composition as in Claim 3, wherein said N-acyl amino acid surfactant is an N-acyl sarcosinate surfactant.

5. A shampoo composition as in Claim 1, 2, 3, or 4, wherein said composition comprises from 5% to 15% of said alkyl ethoxylated sulfate, from 1.5% to 10% of said amphoteric surfactant, and from 0.5% to 5% of said N-acyl amino acid surfactant.

6. A shampoo composition as in Claim 1, 2, 3, 4, or 5 wherein a 30% aqueous solution of said amphoteric surfactant has an absorbance value at 440nm in a 1.0 path length of no greater than 0.035.

7. A shampoo composition as in Claim 1, 2, 3, 4, 5, or 6 wherein said absorbance value of said shampoo is no greater than 0.025 after 30 days at 38°C.

**Patentansprüche**

1. Milde, im wesentlichen farblose Schampoozusammensetzung, dadurch gekennzeichnet, daß sie umfaßt:

   (a) 5 bis 15 Gew.-% eines anionischen ethoxylierten Alkylsulfat-Tensids mit einem durchschnittlichen Ethoxylierungsgrad von mindestens 2,0;
   (b) 2,5 bis 8 Gew.-% eines amphoteren Tensids, gewählt aus der Betain-Tenside, Imidazolin-Tenside, Aminoalkanoat-Tenside und Iminodialkanoat-Tenside und Mischungen hiervon umfassenden Gruppe;
   (c) 0.5 bis 10 Gew.-% eines N-Acylaminosäure-Tensids oder ein Salz hiervon;
   (d) mindestens 0,05 Gew.-% der Zusammensetzung carbonylhaltige Parfümverbindungen; und
   (e) Wasser;

   wobei die Schampoozusammensetzung im wesentlichen frei an anionischen Alkylsulfat-Tensiden, primären Aminen und Ammoniak ist, und die Schampoozusammensetzung einen Extinktionswert bei 440 nm in einer 1,0 cm Weglänge von nicht mehr als 0,030 nach Lagerung bei 38°C während 30 Tagen aufweist, und wobei das Gewichtsverhältnis von (a) zu (b) im Bereich von 10:1 bis 2:1 liegt, und wobei (a) ein Alkali- oder Erdalkalimetallsalz ist.

2. Schampoozusammensetzung nach Anspruch 1, wobei das amphotere Tensid aus der Gruppe gewählt ist, welche besteht aus

   (i) Betain-Tensiden der Formel:

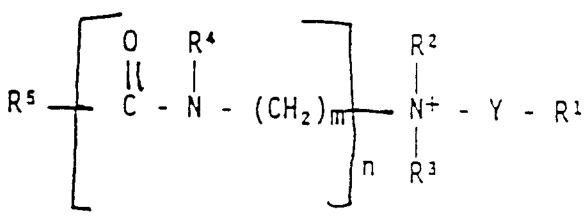

worin $R_1$ ein Vertreter ist, gewählt aus der
COOM und

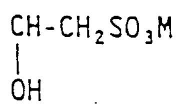

umfassenden Gruppe,
$R_2$ Niederalkyl oder Hydroxyalkyl ist;
$R_3$ Niederalkyl oder Hydroxyalkyl ist;
$R_4$ ein Vertreter ist, gewählt aus der Wasserstoff und Niederalkyl umfassenden Gruppe:
$R_5$ Höheralkyl oder Alkenyl ist;

Y Niederalkyl, vorzugsweise Methyl ist;
m eine ganze Zahl von 2 bis 7 ist;
n die ganze Zahl 1 oder 0 ist;
M Wasserstoff oder ein Kation ist;

(ii) amphoteres Tensid der Formel

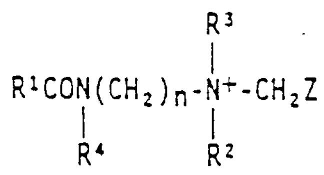

worin $R^1$ $C_8$-$C_{22}$-Alkyl oder -Alkenyl ist, $R^2$ Wasserstoff oder $CH_2CO_2M$ ist, $R^3$ $CH_2CH_2OH$ oder $CH_2CHOCH_2CH_2COOM$ ist, $R^4$ Wasserstoff, $CH_2CH_2OH$ oder $CH_2CH_2OCH_2CH_2COOM$ ist, Z $CO_2M$ oder $CH_2CO_2M$ ist, n 2 oder 3 ist und M Wasserstoff oder ein Kation ist; und
(iii) Aminoalkanoaten der Formel:

$$R\text{-}NH(CH_2)_nCOOM;\ \text{und}$$

Iminodialkanoaten der Formel:

$$R\text{-}N[CH_2)_mCOOM]_2$$

worin n und m 1 bis 4 sind, R $C_8$-$C_{22}$-Alkyl oder -Alkenyl ist, und M Wasserstoff oder Alkali oder Erdalkali mit einem wie vorangehend beschriebenen Metall ist;
(iv) und Mischungen hiervon.

**3.** Schampoozusammensetzung nach Anspruch 1 oder 2, wobei das N-Acylaminosäure-Tensid oder Salz hiervon die Formel

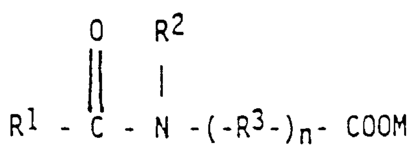

aufweist, worin $R^1$ $C_8$-$C_{24}$-Alkyl oder -Alkenyl ist; $R^2$ -H, $C_1$-$C_4$-Alkyl, Phenyl oder $-CH_2COOM$ ist; $R^3$ $-C-R^4_2$ ist; n 1 bis 4 ist; $R^4$ H, $C_1$-$C_6$-Alkyl oder $C_1$-$C_6$-Alkylester ist; und M H oder ein Kation oder eine Mischung hiervon ist.

**4.** Schampoozusammensetzung nach Anspruch 3, wobei das N-Acylaminosäure-Tensid ein N-Acylsarcosinat-Tensid ist.

**5.** Schampoozusammensetzung nach Anspruch 1, 2, 3 oder 4, wobei die Zusammensetzung 5 bis 15% des ethoxylierten Alkylsulfats, 1,5 bis 10% des amphoteren Tensids und 0,5 bis 5% des N-Acylaminosäure-Tensids umfaßt.

**6.** Schampoozusammensetzung nach Anspruch 1, 2, 3, 4 oder 5, wobei eine 30%-ige wäßrige Lösung des amphoteren Tensids einen Extinktionswert bei 440 nm bei einer 1,0 cm Weglänge von nicht mehr als 0,035 aufweist.

**7.** Schampoozusammensetzung nach Anspruch 1, 2, 3, 4, 5 oder 6, wobei der Extinktionswert des Schampoos nicht größer als 0,025 nach 30 Tagen bei 38°C ist.

## Revendications

1. Composition de shampooing doux, sensiblement incolore, caractérisée en ce qu'elle comprend:

   (a) de 5% à 15%, en poids, d'un tensioactif anionique alkylsulfate éthoxylé ayant un degré moyen d'éthoxylation d'au moins 2,0;
   (b) de 2,5% à 8%, en poids, d'un tensioactif amphotère choisi dans le groupe constitué par les tensioactifs bétaïnes, les tensioactifs imidazolines, les tensioactifs aminoalcanoates et les tensioactifs iminodialcanoates, et leurs mélanges;
   (c) de 0,5% à 10%, en poids, d'un tensioactif N-acylaminoacide, ou d'un de ses sels;
   (d) au moins 0,05%, en poids de la composition, de composés parfums contenant des groupes carbonyle; et
   (e) de l'eau;

   dans laquelle ladite composition de shampooing est sensiblement dépourvue de tensioactif anionique alkylsulfate, d'amines primaires et ammoniaque, et ladite composition de shampooing possède une valeur d'absorbance à 440 nm, sur un trajet de 1,0 cm de longueur, non supérieure à 0,030 après 30 jours d'emmagasinage à 38°C, et dans laquelle le rapport pondéral de (a) à (b) est dans l'intervalle de 10:1 à 2:1, et dans laquelle (a) est un sel de métal alcalin ou alcalino-terreux.

2. Composition de shampooing selon la revendication 1, caractérisée en ce que ledit tensioactif amphotère est choisi dans le groupe constitué par

   (i) les tensioactifs bétaïnes de formule:

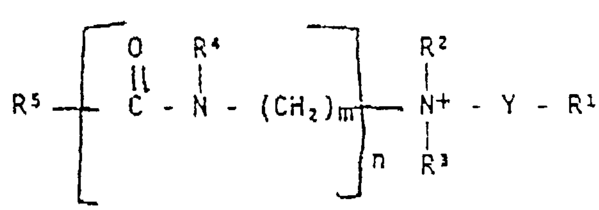

dans laquelle $R_1$ est un membre choisi dans le groupe constitué par COOM et

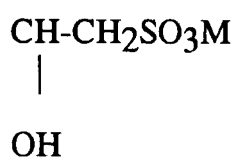

$R_2$ est un groupe alkyle ou hydroxyalkyle inférieur;
$R_3$ est un groupe alkyle ou hydroxyalkyle inférieur;
$R_4$ est un membre choisi dans le groupe constitué par un atome d'hydrogène et un groupe alkyle inférieur;
$R_5$ est un groupe alkyle ou alcényle inférieur;
Y est un groupe alkyle inférieur, de préférence méthyle;
m est un nombre entier de 2 à 7;
n est le nombre entier 1 ou 0;
M est un atome d'hydrogène ou un cation;

   (ii) un tensioactif amphotère de formule

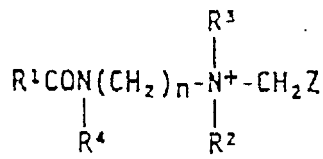

dans laquelle $R^1$ est un groupe alkyle ou alcényle en $C_8$-$C_{22}$, $R^2$ est un atome d'hydrogène ou $CH_2CO_2M$, $R^3$ est $CH_2CH_2OH$ ou $CH_2CHOCH_2CH_2COOM$, $R^4$ est un atome d'hydrogène, $CH_2CH_2OH$ ou $CH_2CH_2OCH_2CH_2COOM$, Z est $CO_2M$ ou $CH_2CO_2M$, n vaut 2 ou 3, et M est un atome d'hydrogène ou un cation; et

(iii) les aminoalcanoates de formule:

$$R\text{-}NH(CH_2)_nCOOM\text{; et}$$

les iminodialcanoates de formule:

$$R\text{-}N[(CH_2)_mCOOM]_2$$

dans laquelle n et m valent de 1 à 4, R est un groupe alkyle ou alcényle en $C_8$-$C_{22}$, et M est un atome d'hydrogène ou un métal alcalin ou alcalino-terreux comme décrit ci-dessus,

(iv) et leurs mélanges.

**3.** Composition de shampooing selon la revendication 1 ou 2, caractérisée en ce que ledit tensioactif N-acylaminoacide, ou son sel, a pour formule:

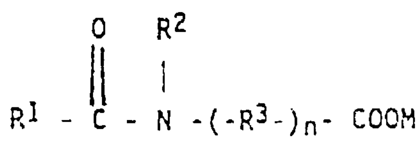

dans laquelle $R^1$ est un groupe alkyle ou alcényle en $C_8$-$C_{24}$;
$R^2$ est -H ou un groupe alkyle en $C_1$-$C_4$, phényle ou - $CH_2COOM$;
$R^3$ est -C-$R^4_2$; n vaut de 1 à 4; $R^4$ est H ou un groupe alkyle en $C_1$-$C_6$ ou ester d'alkyle en $C_1$-$C_6$; et M est H ou un cation, ou un mélange de ceux-ci.

**4.** Composition de shampooing selon la revendication 3, caractérisée en ce que ledit tensioactif N-acylaminoacide est un tensioactif N-acylsarcosinate.

**5.** Composition de shampooing selon la revendication 1, 2, 3 ou 4, caractérisée en ce que ladite composition comprend de 5% à 15% dudit alkylsulfate éthoxylé, de 1,5% à 10% dudit tensioactif amphotère et de 0,5% à 5% dudit tensioactif N-acylaminoacide.

**6.** Composition de shampooing selon la revendication 1, 2, 3, 4 ou 5, caractérisée en ce qu'une solution aqueuse à 30% dudit tensioactif amphotère possède une valeur d'absorbance à 440 nm, sur un trajet de 1,0 cm de longueur, non supérieure à 0,035.

**7.** Composition de shampooing selon la revendication 1, 2, 3, 4, 5 ou 6, caractérisée en ce que ladite valeur d'absorbance dudit shampooing n'est pas supérieure à 0,025 après 30 jours à 38°C.